# EUROPÄISCHE PATENTSCHRIFT

(11) **EP 1 007 014 B1**
(45) Veröffentlichungstag und Bekanntmachung des Hinweises auf die Patenterteilung: **02.05.2003**
(21) Anmeldenummer: 98901337.0
(22) Anmeldetag: 03.01.1998
(51) Int. Cl.: A61K 9/70

(54) **TRANSDERMALES THERAPEUTISCHES SYSTEM ZUR ABGABE VON HORMONEN**
TRANSDERMAL, HORMONE-DELIVERING THERAPEUTIC SYSTEM
SYSTEME THERAPEUTIQUE TRANSDERMIQUE POUR ADMINISTRER DES HORMONES

(30) Priorität: 14.01.1997 DE 19700913
(43) Veröffentlichungstag der Anmeldung: 14.06.2000
(73) Patentinhaber: LTS Lohmann Therapie-Systeme AG, 56626 Andernach (DE)
(72) Erfinder: MECONI, Reinhold, D-56567 Neuwied (DE); SEIBERTZ, Frank, D-53557 Bad Hönningen (DE)
(74) Vertreter: Flaccus, Rolf-Dieter, Dr.
(86) Internationale Anmeldenummer: EP9800014
(87) Internationale Veröffentlichungsnummer: WO98031349

(56) Entgegenhaltungen:
- WO-A-96/21433
- US-A- 5 158 734

## Beschreibung

Die Erfindung bezieht sich auf ein wirkstoffhaltiges Pflaster zur kontrollierten Abgabe von Estradiol oder seinen pharmazeutisch unbedenklichen Derivate, allein oder in Kombination mit Gestagenen an die menschliche oder tierische Haut, mit einem wirkstoffhaltigen Reservoir auf der Basis von Ethylcellulose und Klebharze aufweisenden Haftklebern.

Transdermale therapeutische Systeme sind seit längerer Zeit bekannt, insbesondere auch für die transdermale Verabreichung von Hormonen, speziell Östrogenen, gegebenenfalls in Kombination mit Gestagenen.

Die Wirkstoffe werden dazu in einem Wirkstoffreservoir auf der Basis von Haftklebern vorgesehen, deren Klebrigkeit einen ausreichenden Hautkontakt bei Applikation ohne übermäßige Erweichung gewährleisten muß, ohne daß Hautirritationen auftreten und deren Wirkstoffaufnahme und -freigabe der gewünschten anhaltenden systemischen Wirkstoffzufuhr über die Haut entsprechen muß.

Als Basis für Estradiol enthaltende Reservoirschichten wurden unter anderem bereits unterschiedliche, insbesondere Ethanol enthaltende Massen auf Kautschuk- oder Polyacrylatbasis vorgesehen (z.B. DE-OS 32 05 258 und EP 0 275 716).

In der DE-OS 39 33 460 werden Wirkstoffpflaster auf der Basis von Homo- und Copolymeren mit mindestens einem Derivat der Acryl- oder Methacrylsäure beschrieben, die zusätzlich in Wasser quellbare Substanzen enthalten sollen.

Aus der DE-PS 40 20 144 ist ein gegebenenfalls auch hormonhaltiges transdermales System mit mindestens einer selbstklebenden Wirkstoffschicht bekannt, die neben Polyacrylat und einem damit verträglichen Filmbildner einen weiten Mengenbereich weichmachender Wirk- und Hilfsstoffe aufweisen soll.

Ein kürzlich beschriebenes transdermales therapeutisches System (siehe DE-OS 195 00 662) umfaßt ein Estradiol und gegebenenfalls Gestagene enthaltendes Wirkstoffreservoir auf der Basis von Ethylcellulose mit einem hohen Anteil an Estern von gegebenenfalls hydriertem Kolophonium als klebrigmachendem Harz zusammen mit bis zu 20 Gew.-% Laurinsäure, die einer Rekristallisation des Wirkstoffs und damit Minderung seiner Freisetzungsrate entgegenwirken soll.

Überraschenderweise wurde nun gefunden, daß eine unerwartet hohe Wirkstofffreisetzung von Estradiol und insbesondere Norethindronacetat aus einem Wirkstoffreservoir auf der Basis von Ethylcellulose und klebrigmachendem Harz erreicht werden kann, das mit einem außergewöhnlich hohen Weichmacheranteil versehen ist.

Das erfindungsgemäße Pflaster der eingangs genannten Art ist demgemäß dadurch gekennzeichnet, daß der Haftkleber einen hohen Weichmacheranteil aufweist.

Insbesondere enthält der Haftkleber zumindest 15 Gew.-%, vorzugsweise zumindest 20 Gew.-% Weichmacher.

Im übrigen kann das wirkstoffhaltige Pflaster Emulgatoren, Antioxidantien und Absorptionsverbesserer enthalten.

Ein solches Pflaster ist insbesondere für eine hohe kombinierte Freisetzung von sowohl Estradiol als auch Norethindronacetat einsetzbar.

Bevorzugte Formulierungen solcher hormonhaltigen Haftklebermassen gemäß der Erfindung zeigen folgende Zusammensetzung (in Gew.%):

| | |
|---|---|
| Ethylcellulose | 5-25 % |
| Klebrigmachende Harze | 10-70 % |
| Weichmacher/Emulgatoren | 20-40 % |
| Estradiol | 1-10 % |
| Norethindronacetat und/oder andere Gestagene | 1-15 % |

Zwar ist die Zugabe von Weichmachern zu wirkstoffhaltigen Haftklebermassen an sich bekannt, wie z.B. auch gemäß der genannten DE-OS 40 20 144, jedoch ist ein Weichmacherzusatz zu den üblichen Haftklebern auf der Basis von Polyacrylaten, Blockcopolymeren und Polyisobutylenen nur begrenzt möglich, da ab einer bestimmten Konzentration die Kohäsion der Haftkleber nachläßt und sie damit für eine transdermale Anwendung ungeeignet werden.

Daß gerade Massen auf Ethylcellulose/Klebharz-Basis mit ungewöhnlich hohen Weichmacherzusätzen besonders nützliche transdermale therapeutische Systeme mit erhöhter Hautpenetration der Hormone und besonders geringer Rekristallisationstendenz derselben bei guten Trageigenschaften des TTS ergeben würden, war keinesfalls vorherzusehen, wie auch die genannte, erst kürzlich bekannt gewordene TTS-Entwicklung mit einem Wirkstoffreservoir auf Ethylcellulose/Klebharz-Basis zeigt, das als Zusatz Laurinsäure und nicht -wie nunmehr - hohe Weichmacheranteile aufweist.

Die erfindungsgemäße Zusammensetzung des Wirkstoffreservoirs auf Ethylcellulose/Klebharz-Basis mit hohem Weichmacheranteil, aber ohne Laurinsäure, zeigt eine gegenüber den Massen gemäß der DE-OS 195 00 662 deutlich erhöhte Hormonfreisetzung, insbesondere bei Einarbeitung einer Kombination von Estradiol und Norethindronacetat.

Dies wird durch die nachfolgend mitgeteilten Ergebnisse (Tabellen 1 und 2) veranschaulicht, von denen Tabelle 1 die Zusammensetzung der untersuchten Massen und Tabelle 2 die Ergebnisse von Versuchsreihen zur Wirkstofffreisetzung zeigt.

Es ist zu ersehen, daß durch den erfindungsgemäßen Weichmachergehalt eine deutliche Verbesserung der Wirkstofffreisetzung und -penetration erreicht wird.

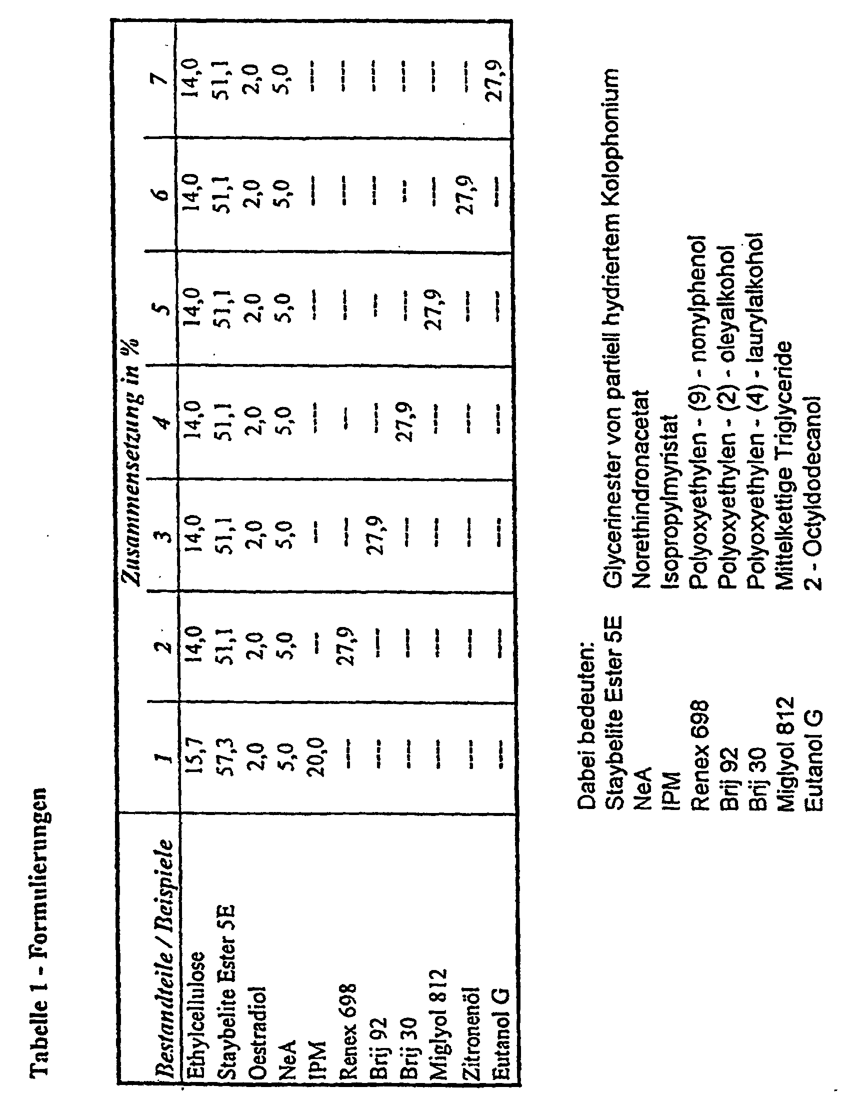

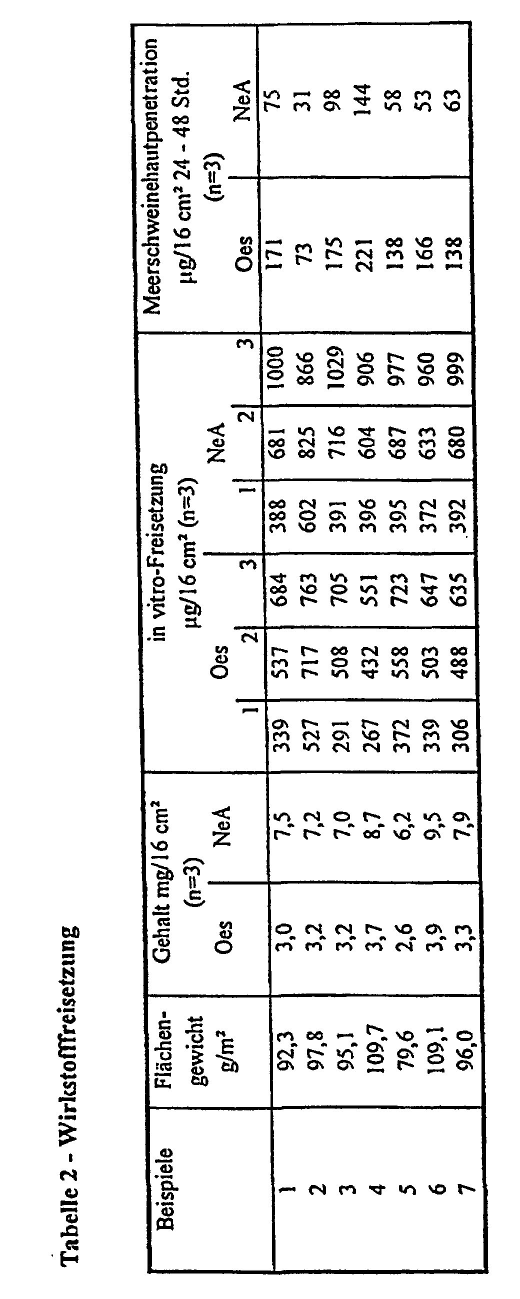

## Patentansprüche

1. Wirkstoffhaltiges Pflaster zur kontrollierten Abgabe von Estradiol oder seinen pharmazeutisch unbedenklichen Derivaten, allein oder in Kombination mit Gestagenen, an die menschliche oder tierische Haut, mit einem wirkstoffhaltigen Reservoir auf der Basis von Ethylcellulose und Klebharze aufweisenden Haftklebern, **gekennzeichnet durch** einen Haftkleber mit einem Weichmacheranteil von zumindest 15 Gew.-%.

2. Pflaster nach Anspruch 1, **gekennzeichnet durch** einen Weichmacheranteil von 20-40 Gew.-%.

3. Pflaster nach Anspruch 1 oder 2, **gekennzeichnet durch** Ester von gegebenenfalls hydriertem Kolophonium als klebrigmachendes Harz.

4. Pflaster nach einem der vorangehenden Ansprüche, **gekennzeichnet durch** einen Emulgator-Anteil im Haftkleber von zumindest 15 Gew.-%, insbesondere von zumindest 20 Gew.-%.

5. Pflaster nach einem der vorangehenden Ansprüche, **gekennzeichnet durch** folgende Zusammensetzung des wirkstoffhaltigen Haftklebers:
| | |
|---|---|
| 5- 25 Gew.-% | Ethylcellulose |
| 10- 70 Gew.-% | klebrigmachende Harze |
| 20- 40 Gew.-% | Weichmacher/Emulgatoren |
| 1- 10 Gew.-% | Estradiol-Anteil |
| 1- 15 Gew.-% | Norethindronacetat und/oder |
| | andere Gestagene. |

6. Pflaster nach einem der vorangehenden Ansprüche, **gekennzeichnet durch** einen Gehalt an Antioxidantien und/oder Absorptionsverbesserern im Haftkleber.

## Claims

1. Active compound-containing patch for the controlled delivery of oestradiol or its pharmaceutically acceptable derivatives, alone or in combination with gestagens, to human or animal skin, having an active compound-containing reservoir based on ethylcellulose and contact adhesives comprising adhesive resins, **characterized by** a contact adhesive having a proportion of plasticizer of at least 15% by weight.

2. Patch according to Claim 1, **characterized by** a proportion of plasticizer of 20-40% by weight.

3. Patch according to claim 1 or 2, **characterized by** esters of optionally hydrogenated colophony as a tackifying resin.

4. Patch according to one of the preceding claims, **characterized by** an emulsifier content in the contact adhesive of at least 15% by weight, in particular of at least 20% by weight.

5. Patch according to one of the preceding claims, **characterized by** the following composition of the active compound-containing contact adhesive:
| | |
|---|---|
| 5-25% by weight of | ethylcellulose |
| 10-70% by weight of | tackifying resins |
| 20-40% by weight of | plasticizer/emulsifiers |
| 1-10% by weight of | oestradiol content |
| 1-15% by weight of | norethindrone acetate and/or |
| | other gestagens. |

6. Patch according to one of the preceding claims, **characterized by** a content of antioxidants and/or absorption enhancers in the contact adhesive.

## Revendications

1. Emplâtre contenant des principes actifs pour la diffusion contrôlée d'estradiol ou de ses dérivés sans risque au plan pharmaceutique, seul ou en combinaison avec des gestagènes, à la peau humaine ou animale, comprenant un réservoir contenant des principes actifs sur la base d'adhésifs de contact présentant de l'éthylcellulose et des résines adhésives, **caractérisé par** un adhésif de contact avec une fraction de plastifiant d'au moins 15 % en poids.

2. Emplâtre selon la revendication 1, **caractérisé par** une fraction de plastifiant de 20 à 40 % en poids.

3. Emplâtre selon les revendications 1 ou 2, **caractérisé par** des esters de colophone hydrogénée le cas échéant comme résine au pouvoir adhésif.

4. Emplâtre selon l'une des revendications précédentes, **caractérisé par** une fraction d'émulsifiant dans l'adhésif de contact d'au moins 15 % en poids, en particulier d'au moins 20 % en poids.

5. Emplâtre selon l'une des revendications précédentes, **caractérisé par** la composition suivante de l'adhésif de contact contenant des principes actifs :
5 à 25 % en poids d'éthylcellulose
10 à 70 % en poids de résines au pouvoir adhésif
20 à 40 % en poids de plastifiants/émulsifiants
1 à 10 % en poids de fraction d'estradiol
1 à 15 % en poids d'acétate de noréthindrone et/ou d'autres gestagènes.

6. Emplâtre selon l'une des revendications précédentes, **caractérisé par** une teneur en antioxydants et/ou en promoteurs d'absorption dans l'adhésif de contact.
